# EUROPEAN PATENT APPLICATION

(11) **EP 4 473 956 A1**
(43) Date of publication of application: **11.12.2024**
(21) Application number: 23749560.1
(22) Date of filing: 20.01.2023
(51) Int. Cl.: A61K 8/25, A61K 8/06, A61K 8/19, A61K 8/27, A61K 8/29, A61K 8/37, A61K 8/73, A61K 8/92, A61Q 1/00, A61Q 17/04

(54) **WATER-IN-OIL EMULSION COSMETIC**

(30) Priority: 02.02.2022 JP 2022015228
(71) Applicant: Shiseido Company, Ltd., Chuo-ku Tokyo 104-0061 (JP)
(72) Inventor: NISHI, Haruka, Tokyo 104-0061 (JP); NISHIDA, Keita, Tokyo 104-0061 (JP)
(74) Representative: Ter Meer Steinmeister & Partner
(86) International application number: PCT/JP2023/001787
(87) International publication number: WO 2023/149244

(57) **Abstract**

[Problem] An objective of the present invention is to provide a water-in-oil emulsion cosmetic lacking powdery squeakiness and having smooth properties in used. [Solution] The water-in-oil emulsion cosmetic of the present invention is characterized by comprising (A) a hydrophobized silica and (B) a semi-solid oil.

## Description

### TECHNICAL FIELD

The present invention relates to a water-in-oil emulsion cosmetic that does not have powdery squeakiness and that has smooth properties in use.

### BACKGROUND ART

In the cosmetics field, various types of powder components, such as synthetic polymer powders and inorganic powders, are blended for the purpose of adjusting the properties in use (spreadability, adhesiveness) of products. In recent years, the use of inorganic powders has been favored due to concerns relating to environmental issues.

Inorganic powders for adjusting the properties in use of a product are also called extender pigments and include mineral-derived mica, sericite, silica (silicic anhydride), etc. In particular, silica is favorably used for having advantages such as being inexpensive and having been confirmed to be very safe to the human body, as well as improving the texture in use of the cosmetic by suppressing stickiness caused by oils used in cosmetics containing high concentrations of oils.

However, when trying to improve the texture in use by using silica, there are cases in which a large blended amount is needed in comparison with synthetic polymer powders, etc. If the blended amount of a powder included in a cosmetic was too high, there were problems in that the flowability of the coating film was reduced so that powdery squeakiness (a texture in which smoothness is gradually lost) occurred over time and the formulation stability was poor.

For example, Patent Document 1 proposes to improve the stability over time of the emulsion state, the texture and the properties in use by using a hydrophobized silica having surfaces treated with dimethylpolysiloxane as an emulsion stabilizer and by using a specific silicone-based surfactant as an emulsifier.

However, although the water-in-oil emulsion composition in the above-mentioned Patent Document 1 has an improved texture in use in that the stickiness that is characteristic of water-in-oil emulsions is suppressed, it was not fully satisfactory in terms of smoothness of the properties in use. Additionally, the properties in use were not considered for the case in which a high concentration of powder components such as extender pigments and ultraviolet scattering agents were blended.

### RELATED ART

### PATENT DOCUMENTS

Patent Document 1: JP 3403223 B

### SUMMARY OF THE INVENTION

### PROBLEM TO BE SOLVED BY THE INVENTION

An objective of the present invention is to provide a water-in-oil emulsion cosmetic in which the texture in use of the cosmetic is improved by using a hydrophobized silica, wherein the water-in-oil emulsion cosmetic does not have powdery squeakiness in a coating film and has smooth properties in use.

### MEANS FOR SOLVING THE PROBLEM

The inventors carried out diligent investigations towards solving the aforementioned problem, as a result of which they discovered that, in a water-in-oil emulsion cosmetic in which the texture in use is improved by using a hydrophobized silica, blending a semi-solid oil results in a water-in-oil emulsion cosmetic that lacks powdery squeakiness in the coating film and that has smooth properties in use, thereby completing the present invention.

The present invention provides a water-in-oil emulsion cosmetic comprising:
(A) a hydrophobized silica; and
(B) a semi-solid oil.

### EFFECTS OF THE INVENTION

The cosmetic according to the present invention, by having the above-mentioned features, can provide a water-in-oil emulsion cosmetic lacking powdery squeakiness in the coating film and having smooth properties in use, even in the case in which a large amount of a powder component such as a hydrophobized silica or an ultraviolet scattering agent is blended.

The "powdery squeakiness" in the present specification refers to a sense of powderiness that occurs after application, with reduced flowability in the coating film and loss of smoothness over time.

### MODES FOR CARRYING OUT THE INVENTION

The water-in-oil emulsion cosmetic according to the present invention is characterized by comprising (A) a hydrophobized silica and (B) a semi-solid oil. Additionally, in another embodiment, the water-in-oil emulsion cosmetic according to the present invention is characterized by comprising (A) a hydrophobized silica, (B) a semi-solid oil and (C) an ultraviolet scattering agent. Hereinafter, the respective components constituting the cosmetic of the present invention will be explained in detail.

### <(A) Hydrophobized silica>

The (A) hydrophobized silica (hereinafter sometimes referred to simply as "component (A)") blended into the cosmetic according to the present invention refers to a silica (silicic anhydride) that is normally used in the cosmetics field, that has a hydrophobic particle surface, and that has an average particle size of 1 to 10 µm, a specific surface area of 200 to 300 m²/g, and that has an oil absorption rate of 100 to 200 ml/100 g. In particular, a porous spherical silica is preferable.

The method for hydrophobizing the particle surfaces of the (A) hydrophobized silica is not particularly limited. However, it is particularly preferable to use a silicone such as methylhydrogen polysiloxane or dimethyl polysiloxane, or a fatty acid ester such as dextrin palmitate as the hydrophobizing agent. Such hydrophobized silicas include, for example, SA-SB-150, SA-SB-300 (manufactured by Miyoshi Kasei, Inc.), etc.

The blended amount of the component (A) is the amount needed in order to provide the cosmetic with a good texture in use, and is not particularly limited. However, the lower limit value is 5% by mass or more, preferably 8% by mass or more relative to the overall amount of the cosmetic, and the upper limit value is 20% by mass or less. The blended amount range may be 5% to 20% by mass, or 8% to 20% by mass. If the blended amount in the cosmetic is less than 5% by mass, there are cases in which sufficient improvement of the texture in use cannot be obtained, such as the cosmetic being sticky, and if the blended amount is more than 20% by mass, there are cases in which the stability becomes poor.

### <(B) Semi-solid oil>

The (B) semi-solid oil (hereinafter sometimes referred to simply as "component (B)") used in the present invention refers to an oil that is normally used in the cosmetics field, that does not exhibit flowability at ambient temperature (25 °C), and that has a melting point of 30 °C to 60 °C, preferably 30 °C to 45 °C, at 1 atm.

Specific examples of the (B) semi-solid oil include vaseline, pentaerythrityl tetra(behenate/benzoate/ethylhexanoate), dipentaerythrityl hexahydroxystearate, phytosteryl macadamiate, dimer dilinoleic acid ester, macadamia nut oil polyglyceryl-6 esters behenate, dipentaerythrityl tetra(hydroxystearate/isostearate), bis-diglyceryl polyacyladipate-2, etc. In particular, pentaerythrityl tetra(behenate/benzoate/ethylhexanoate) or dipentaerythrityl hexahydroxystearate is preferably used.

The blended amount of component (B) is 1% to 5% by mass, preferably 1.5% to 4% by mass relative to the overall amount of the cosmetic. If the blended amount of the cosmetic is less than 1% by mass, there are cases in which sufficient stability for use as a cosmetic cannot be obtained, and if the blended amount is more than 5% by mass, there are cases in which the cosmetic is lacking in spreadability.

Additionally, the water-in-oil emulsion cosmetic according to the present invention has a viscosity of 10,000 mPa·s or higher, preferably 30,000 mPa·s or higher, and even more preferably 45,000 mPa s or higher. The upper limit value of the viscosity is preferably 100,000 mPa·s or lower. Thus, a suitable viscosity range is 10,000 to 100,000 mPa·s, more suitably 45,000 to 100,000 mPa·s. If the viscosity is lower than 10,000 mPa·s, there are cases in which the oil undergoes syneresis. Meanwhile, if the viscosity is higher than 100,000 mPa s, the viscosity becomes too high and the cosmetic becomes heavy to spread at the time of application, and good properties in use cannot be obtained. The viscosity in the present specification is a value measured by a B-type viscometer at 30 °C.

### <(C) Ultraviolet scattering agent>

The water-in-oil emulsion cosmetic according to the present invention may further have (C) an ultraviolet scattering agent blended therein.

The (C) ultraviolet scattering agent (hereinafter sometimes referred to simply as "component (C)") blended into the cosmetic according to the present invention is not particularly limited as long as it is a powder that is used as an ultraviolet scattering agent in the cosmetics field. Specific examples include one or more types selected from among titanium oxide, zinc oxide, barium sulfate, iron oxide, talc, mica, sericite, kaolin, titanated mica, Prussian blue, chromium oxide, chromium hydroxide, silica, cerium oxide, etc. In particular, a powder having a refractive index of 1.5 or higher, for example, zinc oxide or titanium oxide, is preferable in terms of optical properties. Additionally, the powder preferably consists of fine particles having an average particle size smaller than 0.1 µm. The lower limit of the average particle size is not particularly limited, but should normally be approximately 5 nm.

Additionally, the (C) ultraviolet scattering agent may have particle surfaces that have undergone a hydrophobization treatment. Due to a surface hydrophobization treatment, the dispersibility in oil and the water resistance improve. Methods of surface treatment include silicone treatments with silicones methylhydrogen polysiloxane, methyl polysiloxane, etc.; alkyl silane treatments; fluorine treatments with perfluoroalkyl phosphoric acid esters, perfluoroalcohols, etc.; amino acid treatments with N-acylglutamic acid, etc.; as well as lecithin treatments; metal soap treatments; fatty acid treatments; alkyl phosphoric acid ester treatments; etc.

The blended amount of component (C) is the amount necessary to obtain the desired ultraviolet protection effects and is not particularly limited. However, the blended amount should normally be 1% by mass or more, for example, 5% to 30% by mass, preferably 10% to 30% by mass relative to the overall amount of the cosmetic. If the blended amount is less than 1% by mass, sufficient ultraviolet protection effects become difficult to obtain, and if the blended amount is more than 30% by mass, the stability tends to become worse.

In the case in which an ultraviolet scattering agent is to be blended into the water-in-oil emulsion cosmetic of the present invention, the lower limit value of the total blended amount of the (C) ultraviolet scattering agent and the (A) hydrophobized silica is 15% by mass or more, preferably 20% by mass or more, relative to the overall amount of the cosmetic, and the upper limit value is 40% by mass or less, preferably 35% by mass or less. The blended amount range may be 15% to 40% by mass, 20% to 35% by mass, etc. In the cosmetic of the present invention, powdery squeakiness does not tend to occur in the coating film, even when a high concentration of powders is blended. Thus, more powders can be blended than normal.

In a water-in-oil emulsion cosmetic, when the amount of powders blended into the oil phase becomes large, there are cases in which syneresis of the oils constituting the external phase occurs over time, and when the oil phase includes pigments, color streaks may be formed due to the pigments becoming non-uniform.

In the water-in-oil emulsion cosmetic according to the present invention, (D) an oil phase thickener comprising (D-1) an organically modified clay mineral and (D-2) a specific oil gelling agent may be further blended in addition to the above-mentioned components (A), (B) and (C) from the aspect of further improving the formulation stability over time. By blending component (D) in the present invention, syneresis of the oils over time can be suppressed, and also, the powder dispersibility can be improved, thereby suppressing the formation of color streaks.

The (D) oil phase thickener that may be blended into the cosmetic according to the present invention can adjust the viscosity of the oil phase, and is a combination of (D-1) an organically modified clay mineral (hereinafter sometimes referred to simply as "component (D-1)") and (D-2) one or more types of oil gelling agents selected from among dextrin fatty acid esters, hydrocarbon-styrene copolymer gelling agents, polymer compounds having urethane backbones, glyceryl fatty acid esters, amino acid gelling agents and sucrose fatty acid esters (hereinafter sometimes referred to simply as "component (D-2)").

As the (D-1) organically modified clay mineral, it is possible to use a clay mineral represented by the following general formula (1), which is a type of colloidal hydrated aluminum silicate having a three-layered structure, modified by a quaternary ammonium salt type cationic surfactant.

(X,Y)₂₋₃(Si,Al)₄O₁₀(OH)₂Z_{1/3}·nH₂O (1)

where X = Al, Fe(III), Mn(III) or Cr(III); Y = Mg, Fe(II), Ni, Zn or Li; and Z = K, Na or Ca.

Specifically, the organically modified clay mineral can be obtained by treating, with a quaternary ammonium salt type cationic surfactant, a clay mineral which may be a natural or synthetic (in this case, an (OH) group in the formula is substituted with a fluorine) clay mineral in the montmorillonite group, such as montmorillonite, saponite or hectorite (commercial products include Veegum, Kunipia, Laponite, etc.), and sodium silicic mica or a synthetic mica known under the name of sodium or lithium taeniolite (commercial products include Dimonite, manufactured by Topy Industries, Ltd., etc.).

The quaternary ammonium salt type cationic surfactant used in this case is represented by the following general formula (2): 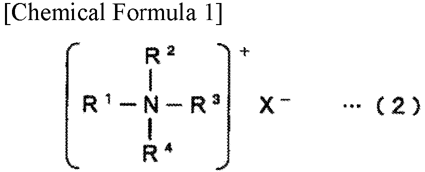 where R¹ represents a benzyl group or an alkyl group having 10 to 22 carbon atoms, R² represents an alkyl group having 10 to 22 carbon atoms or a methyl group, R³ and R⁴ represent hydroxyalkyl groups or alkyl groups having 1 to 3 carbon atoms, and X represents a halogen atom or a methylsulfate residue.

Examples of the quaternary ammonium salt type cationic surfactant include dodecyl trimethyl ammonium chloride, myristyl trimethyl ammonium chloride, cetyl trimethyl ammonium chloride, stearyl trimethyl ammonium chloride, arachyl trimethyl ammonium chloride, behenyl trimethyl ammonium chloride, myristyl dimethyl ethyl ammonium chloride, cetyl dimethyl ethyl ammonium chloride, stearyl dimethyl ethyl ammonium chloride, arachyl dimethyl ethyl ammonium chloride, behenyl dimethyl ethyl ammonium chloride, myristyl diethyl methyl ammonium chloride, cetyl diethyl methyl ammonium chloride, stearyl diethyl methyl ammonium chloride, arachyl diethyl methyl ammonium chloride, behenyl diethyl methyl ammonium chloride, benzyl dimethyl myristyl ammonium chloride, benzyl dimethyl cetyl ammonium chloride, benzyl dimethyl stearyl ammonium chloride, benzyl dimethyl behenyl ammonium chloride, benzyl methyl ethyl cetyl ammonium chloride, benzyl methyl ethyl stearyl ammonium chloride, dibehenyl dihydroxyethyl ammonium chloride, and corresponding bromides, etc., and further thereto, dipalmityl propyl ethyl ammonium methyl sulfate, etc. When carrying out the present invention, one or more of these compounds may be arbitrarily selected.

Representative examples of component (D-1) include dimethyl distearyl ammonium hectorite (disteardimonium hectorite), dimethyl alkyl ammonium hectorite, benzyl dimethyl stearyl ammonium hectorite, distearyl dimethyl ammonium chloride-treated aluminum-magnesium silicate, etc. Of these, dimethyl distearyl ammonium hectorite is particularly preferred. As commercial products, Bentone 27 (benzyl dimethyl stearyl ammonium chloride-treated hectorite, manufactured by Elementis Japan KK) and Bentone 38 (distearyl dimethyl ammonium chloride-treated hectorite, manufactured by Elementis Japan KK) are preferred.

The dextrin fatty acid esters of component (D-2) are esters of dextrin or reduced dextrin with a higher fatty acid, which can be used without any particular limitations as long as they are commonly used in cosmetics. A dextrin or a reduced dextrin having an average degree of polymerization of 3 to 100 is preferably used. Additionally, as the constituent fatty acids of the dextrin fatty acid esters, saturated fatty acids having 8 to 22 carbon atoms are preferably used. Specific examples include dextrin palmitate, dextrin oleate, dextrin stearate, dextrin myristate, dextrin (palmitate/2-ethylhexanoate), etc.

The hydrocarbon-styrene copolymer gelling agents of component (D-2) refer to copolymers of at least one styrene unit with a unit selected from among butadiene, ethylene, propylene, butylene and isopropene, hydrogenated hydrocarbon copolymers, and mixtures thereof. Specific examples include mixtures of (ethylene/propylene/styrene) copolymers and (butylene/ethylene/styrene) copolymers. An example of a commercially available product is Versagel ME 2000 (manufactured by Calumet Penreco LLC).

Examples of the polymer compounds having urethane backbones of component (D-2) include castor oil/IPDI copolymer, which is a copolymer of castor oil with isophorone diisocyanate (IPDI), etc. An example of a commercially available product is Estogel M (castor oil/IPDI copolymer (dissolved in tri(caprylic/capric)glyceride), manufactured by DKSH).

The glyceryl fatty acid esters of component (D-2) are esterized reaction products obtained by reacting glycerin, a dibasic acid having 18 to 28 carbon atoms, and a fatty acid having 8 to 28 carbon atoms (excluding dibasic acids), which can be used without any particular limitations as long as it can be generally used in cosmetics. Specific examples include glyceryl (behenate/isostearate/eicosanedioate), glyceryl (behenate/eicosanedioate), polyglyceryl-10 (behenate/eicosanedioate), etc.

Examples of the amino acid gelling agents of component (D-2) include N-lauroyl-L-glutamic acid dibutylamide (dibutyl lauroyl glutamide), N-2-ethylhexanoyl-L-glutamic acid dibutylamide (dibutyl ethylhexanoyl glutamide), polyamide-8, polyamide-3, etc.

As examples of the sucrose fatty acid esters of component (D-2), those in which the fatty acids are linear or branched, saturated or unsaturated, and have 12 to 22 carbon atoms can be favorably used. Specific examples include sucrose caprylic acid esters, sucrose capric acid esters, sucrose lauric acid esters, sucrose myristic acid esters, sucrose palmitic acid esters, sucrose stearic acid esters, sucrose oleic acid esters, sucrose erucic acid esters, etc.

The (D) oil phase thickener is an optional blended component in the cosmetic of the present invention and therefore does not essentially need to be blended. However, when blended, it should preferably be blended in an amount allowing the effects of the blending to be observed, while also being within limits in which the blended amount does not become excessive and problems such as the feel in use becoming poor are not observed. A suitable blended amount of the (D-1) organically modified clay mineral in the cosmetic according to the present invention should preferably be approximately 0.2% to 1.7% by mass relative to the overall amount of the cosmetic, and a suitable blended amount of the (D-2) specific oil gelling agent should preferably be approximately 0.1% to 3% by mass relative to the overall amount of the cosmetic.

Additionally, when blending a pigment into a water-in-oil emulsion cosmetic, it is common to perform a surface hydrophobization treatment.

In the water-in-oil emulsion cosmetic of the present invention, an inorganic pigment surface-treated with a surface treatment agent containing an amino acid or a salt thereof ((E) amino acid-treated pigment) may be used from the aspect of further improving the formulation stability over time.

The (E) amino acid-treated pigment (hereinafter sometimes referred to as "component (E)") that may be blended into the cosmetic according to the present invention refers to an inorganic pigment that is surface-treated with a surface treatment agent containing an amino acid or a salt thereof. By using a specific compound as the surface treatment agent, a cosmetic with excellent powder dispersibility is obtained. As the surface treatment agent for the pigment in the present invention, the amino acids mentioned below or salts thereof may be used, or another compound that is generally used as a surface treatment agent for powders may be further included in addition to the amino acids mentioned below or salts thereof.

Specific examples of the pigments include red iron oxide (Bengal red), iron titanate, γ-iron oxide, yellow iron oxide, ocher, black iron oxide, carbon black, lower titanium oxides, mango violet, cobalt violet, chromium oxide, chromium hydroxide, cobalt titanate, ultramarine blue, Prussian blue, etc. In particular, a pigment-grade iron oxide such as yellow iron oxide, red iron oxide or black iron oxide, a pigment-grade titanium oxide, etc. is preferably used as the pigment in the present invention. In this case, pigment grade means that the average particle size is 100 nm to 1 µm.

Examples of the "amino acid" used as the surface treatment agent for component (E) include glutamic acid, aspartic acid, lysine etc., among which glutamic acid and aspartic acid are preferred. Additionally, the "amino acid" may be an "amino acid acylated by a saturated fatty acid" in which an acyl group, preferably a saturated fatty acid having 12 to 20 carbon atoms, is condensed on an amino group in the amino acid. Examples of the "acyl group" include stearoyl groups, lauroyl groups, etc. The "salt" may be selected from among alkali metal salts such as sodium and potassium, and alkaline earth metal salts, among which sodium salts are preferred. Specific examples of acylated amino acids include disodium stearoyl glutamate, sodium lauroyl glutamate, sodium lauroyl aspartate and lauroyl lysine.

The (E) amino acid-treated pigment in the present invention can be prepared by a conventional method, by adsorbing an amino acid or a salt thereof to the pigment surfaces.

The surface treatment agent in the (E) amino acid-treated pigment used in the present invention is preferably a surface treatment agent containing an amino acid selected from between disodium stearoyl glutamate and sodium lauroyl glutamate, and is more preferably a surface treatment agent containing disodium stearoyl glutamate.

A commercially available product may be used as the (E) amino acid-treated pigment in the present invention, and preferable commercially available products include, for example, ASL-treated powders such as ASL-1 TiO2 CR-50, ASL-Red R516P, ASL-Yellow LL-100P and ASL-Black BL-100P; ASI-treated powders such as ASI TiO2 CR-50, ASI-Red R516P, ASI-Yellow LL-100P, ASI- Black BL-100P and ASI-Talc JA46R (all of the above manufactured by Daito Kasei Kogyo Co., Ltd.); and NHS-treated powders such as NHS-Titanium CR-50, NHS-Red R516PS, NHS-Yellow LL-100P, NHS-Black BL-100P, NHS-Mica M-102 and NHS-Talc JA-46R (all of the above manufactured by Miyoshi Kasei, Inc.).

As component (E) in the present invention, one or more types of the aforementioned pigments treated with a surface treatment agent may be combined and blended.

Component (E) is an optional blended component in the cosmetic of the present invention and therefore does not essentially need to be blended. However, when blended, it should preferably be blended in an amount allowing the effects of the blending to be observed, while also being within limits in which the blended amount does not become excessive and problems such as the feel in use becoming poor are not observed. A suitable blended amount of component (E) in the cosmetic according to the present invention should preferably be approximately 1% to 8% by mass relative to the overall amount of the cosmetic.

In the cosmetic of the present invention, 5% by mass or more of component (E) may be blended relative to the overall amount of the cosmetic.

In the water-in-oil emulsion cosmetic according to the present invention, by blending the aforementioned components (D) and (E), syneresis of the oils can be suppressed and the powder dispersibility can be improved, thereby resulting in a cosmetic in which the formulation stability over time is even better.

The water blended into the cosmetic according to the present invention is selected, as needed, from among ion-exchanged water, purified water, tap water, natural water, etc. The blended amount is the balance (in percentage by mass relative to the overall amount of the cosmetic) with respect to the sum of the essential components of the present invention and the other optional blended components. In general, the blended amount should preferably be approximately 5% to 40% by mass relative to the overall amount of the cosmetic.

Aside from the components mentioned above, other optional components that are used in external preparations for the skin such as normal cosmetic products and pharmaceutical products may be appropriately blended, as needed, into the cosmetic according to the present invention within a range not compromising the objective and effects of the present invention. The other optional components include, for example, powders other than components (A), (C) and (E); oils; ultraviolet absorbing agents; non-ionic, cationic and silicone-based surfactants; lower alcohols; polyhydric alcohols; chelators; pH adjusters; anti-oxidants; fragrances; preservatives; microbicidal agents; various types of drugs, etc. However, the optional components are not limited to those indicated.

The cosmetic of the present invention can be produced in accordance with a normal method. That is, the water-in-oil emulsion cosmetic can be obtained by mixing the powder components and the oil phase components while heating as needed, thereby obtaining an oil phase component mixture in which the powder components are uniformly dispersed, adding a water phase component mixture, obtained by separately mixing and dissolving the water phase components, to the oil phase component mixture, and emulsifying the mixture with a homomixer, etc.

The cosmetic of the present invention can be prepared in a format such as a paste, a cream or a balm. In particular, the present invention is suited to forming a cream-type cosmetic filling a wide-mouthed container or a tube.

The cosmetic of the present invention may be provided as a makeup cosmetic such as a makeup base, a foundation, a concealer, a blush, an eyeshadow, a mascara, an eyeliner, an eyebrow pencil, an overcoat or a lipstick; or as a skin-care cosmetic such as a sunscreen, among which it is suited to forming a makeup cosmetic having sunscreen effects.

### EXAMPLES

Though the present invention will be explained in further detail by providing examples below, the present invention is not limited in any way by these examples. Where not specially noted, the blended amounts are indicated in percentage by mass relative to the systems in which the relevant components are blended. Before specifically explaining the respective examples, the evaluation methods that were employed will be explained.

### 1. Evaluation of properties in use

Actual usage tests were performed by five expert panelists. The properties in use (smoothness) when each sample was applied to the skin and the lack of powder squeakiness of the coating film were evaluated by assigning scores based on the five-level evaluation indicated below, and by calculating the average values thereof.
5: Very smooth
4: Smoothness of the coating film is slightly reduced
3: Smoothness lost compared to time of application, but no powderiness felt
2: Powderiness felt
1: Very powdery

### (Evaluation criteria)

A: Average score 4.0 or higher
B: Average score 3.0 or higher and lower than 4.0
C: Average score 1.5 or higher and lower than 3.0
D: Average score lower than 1.5

### 2. Evaluation of formulation stability

The cosmetic of each example was placed at rest for four weeks at 50 °C, then visually observed for the presence or absence of syneresis (oil rising), and evaluated based on the evaluation criteria indicated below.

### (Evaluation criteria)

A: No syneresis occurred, with the surface having no roughness and being lustrous
B: No syneresis occurred, but there was some roughness on the surface
C: Small amount of transparent liquid observed on surface (slight syneresis occurred)
D: State of collected transparent liquid observed (significant syneresis occurred)

The foundation creams having the compositions listed in Table 1 below were prepared by conventional methods. The prepared samples were evaluated regarding their properties in use in accordance with the aforementioned evaluation method. The results are also shown in the table.

**[Table 1]**

| Composition | Ex 1 | Ex 2 | Ex 3 | Ex 4 | Ex 5 | Ex 6 | Com p Ex 1 | Com p Ex 2 | Com p Ex 3 |
|---|---|---|---|---|---|---|---|---|---|
| Hydrogenated didecene | 17 | 13 | 13 | 13 | 13 | 13 | 13 | 10 | 10 |
| Hydrogenated polydecene | 1 | 1 | 1 | 1 | - | 1 | 3 | 3 | 3 |
| Caprylyl methicone | 4 | 4 | 4 | 4 | 4 | 5 | 5 | 5 | 5 |
| Pentaerythrityl tetra(behenate/ benzoate/ethylhexanoate) | 3 | 3 | - | - | 4 | 2 | - | - | - |
| Dipentaerythrityl hexahydroxystearate | - | - | 3 | - | - | - | - | - | - |
| Phytosteryl macadamiate | - | - | - | 3 | - | - | - | - | - |
| Isododecane | - | - | - | - | - | - | - | 3 | - |
| Undecane, tridecane | - | - | - | - | - | - | - | - | 3 |
| Ethylhexyl salicylate | 4 | 4 | 4 | 4 | 4 | 4 | 4 | 4 | 4 |
| Diisopropyl sebacate | 4 | 4 | 4 | 4 | 4 | 4 | 4 | 4 | 4 |
| Sorbitan sesquiisostearate | 1 | 1 | 1 | 1 | 1 | 1 | 1 | 1 | 1 |
| Isostearic acid | 1 | 1 | 1 | 1 | 1 | 1 | 1 | 1 | 1 |
| Distearyldimonium chloride | 0.1 | 0.1 | 0.1 | 0.1 | 0.1 | 0.1 | 0.1 | 0.1 | 0.1 |
| Palmitic acid | 0.0 5 | 0.0 5 | 0.0 5 | 0.0 5 | 0.0 5 | 0.0 5 | 0.05 | 0.05 | 0.05 |
| PEG-10 hydrogenated castor oil | 2.5 | 2.5 | 2.5 | 2.5 | 2.5 | 2.5 | 2.5 | 2.5 | 2.5 |
| Lauryl PEG-9 polydimethylsiloxyethyl dimethicone | 3.5 | 3.5 | 3.5 | 3.5 | 3.5 | 3.5 | 3.5 | 3.5 | 3.5 |
| Dextrin palmitate | 1 | 1 | 1 | 1 | 1 | 1 | 1 | 1 | 1 |
| Disteardimonium hectorite | 0.9 | 0.9 | 0.9 | 0.9 | 0.9 | 0.9 | 0.9 | 1.1 | 0.8 |
| Hydrophobized fine-particle titanium oxide | 5 | 5 | 5 | 5 | 5 | 5 | 5 | 5 | 5 |
| Hydrophobized fine-particle zinc oxide | 10 | 10 | 10 | 10 | 10 | 10 | 10 | 10 | 10 |
| Hydrophobized silica | 10 | 10 | 10 | 10 | 10 | 10 | 10 | 10 | 10 |
| Amino acid-treated pigment-grade titanium oxide | - | 4 | 4 | 4 | 4 | 4 | 4 | 4 | 3.6 |
| Amino acid-treated yellow iron oxide | - | 0.9 | 0.9 | 0.9 | 0.9 | 0.9 | 0.9 | 0.9 | 0.9 |
| Amino acid-treated red iron oxide | - | 0.3 5 | 0.3 5 | 0.3 5 | 0.3 5 | 0.3 5 | 0.35 | 0.35 | 0.35 |
| Amino acid-treated black iron oxide | - | 0.1 8 | 0.1 8 | 0.1 8 | 0.1 8 | 0.1 8 | 0.18 | 0.18 | 0.16 |
| Water | 25. 25 | 23. 82 | 23. 82 | 23. 82 | 23. 82 | 23. 82 | 23.8 2 | 23.6 2 | 24.3 4 |
| Alcohol | 2 | 2 | 2 | 2 | 2 | 2 | 2 | 2 | 2 |
| Phenoxyethanol | 0.5 | 0.5 | 0.5 | 0.5 | 0.5 | 0.5 | 0.5 | 0.5 | 0.5 |
| EDTA-3Na | 0.2 | 0.2 | 0.2 | 0.2 | 0.2 | 0.2 | 0.2 | 0.2 | 0.2 |
| Glycerin | 4 | 4 | 4 | 4 | 4 | 4 | 4 | 4 | 4 |
| TOTAL | 100 | 100 | 100 | 100 | 100 | 100 | 100 | 100 | 100 |
| Properties in use (smoothness, lack of powdery squeakiness) | A | A | A | A | A | B | D | D | D |

As indicated in Table 1, the cosmetics of Comparative Example 1, which did not include the (B) semi-solid oil, and Comparative Example 2 and Comparative Example 3, which included liquid oils instead of the (B) semi-solid oil, each had poor properties in use.

Meanwhile, the cosmetics of Examples 1, 2, 5 and 6 including components (A), (B) and (C) of the present invention, and the cosmetics of Examples 3 and 4, in which the types of the (B) semi-solid oil were variously changed, all had smooth properties in use, lacking powdery squeakiness of the coating film.

The foundation creams having the compositions indicated in Table 2 below were prepared by conventional methods. The prepared samples were evaluated regarding formulation stability in accordance with the aforementioned evaluation method. Examples 1 and 2 are the same as those listed in Table 1.

**[Table 2]**

| Composition | Ex 1 | Ex 2 | Com p Ex 4 |
|---|---|---|---|
| Hydrogenated didecene | 17 | 13 | 13 |
| Hydrogenated polydecene | 1 | 1 | 1 |
| Caprylyl methicone | 4 | 4 | 4 |
| Isododecane | - | - | 3 |
| Pentaerythrityl tetra(behenate/benzoate/ethylhexanoate) | 3 | 3 | - |
| Ethylhexyl salicylate | 4 | 4 | 4 |
| Diisopropyl sebacate | 4 | 4 | 4 |
| Sorbitan sesquiisostearate | 1 | 1 | 1 |
| Isostearic acid | 1 | 1 | 1 |
| Distearyldimonium chloride | 0.1 | 0.1 | 0.1 |
| Palmitic acid | 0.05 | 0.05 | 0.05 |
| PEG-10 hydrogenated castor oil | 2.5 | 2.5 | 2.5 |
| Lauryl PEG-9 polydimethylsiloxyethyl dimethicone | 3.5 | 3.5 | 3.5 |
| Dextrin palmitate | 1 | 1 | 1 |
| Disteardimonium hectorite | 0.9 | 0.9 | 0.9 |
| Hydrophobized fine-particle titanium oxide | 5 | 5 | 5 |
| Hydrophobized fine-particle zinc oxide | 10 | 10 | 10 |
| Hydrophobized silica | 10 | 10 | 10 |
| Amino acid-treated pigment-grade titanium oxide | - | 4 | 4 |
| Amino acid-treated yellow iron oxide | - | 0.9 | 0.9 |
| Amino acid-treated red iron oxide | - | 0.35 | 0.35 |
| Amino acid-treated black iron oxide | - | 0.18 | 0.18 |
| Water | 25.2 5 | 23.8 2 | 23.8 2 |
| Alcohol | 2 | 2 | 2 |
| Phenoxyethanol | 0.5 | 0.5 | 0.5 |
| EDTA-3Na | 0.2 | 0.2 | 0.2 |
| Glycerin | 4 | 4 | 4 |
| TOTAL | 100 | 100 | 100 |
| Properties in use (smoothness, lack of powdery squeakiness) | A | A | D |
| Formulation stability | B | A | D |

As indicated in Table 2, the cosmetics indicated in the table contained 25% by mass of the (A) hydrophobized silica and the (C) ultraviolet scattering agent combined. The cosmetic of Comparative Example 4 containing a liquid oil instead of the (B) semi-solid oil of the present invention performed poorly in terms of both properties in use and formulation stability. Meanwhile, the cosmetics of Examples 1 and 2, which are cosmetics containing components (A), (B) and (C) of the present invention, performed well in terms of both properties in use and formulation stability. Additionally, it was demonstrated that the formulation stability further improves in the case in which the (E) amino acid-treated pigment of the present invention is included.

## Claims

1. A water-in-oil emulsion cosmetic comprising:
(A) a hydrophobized silica; and
(B) a semi-solid oil.

2. The water-in-oil emulsion cosmetic according to claim 1, wherein the blended amount of the component (A) is 5% by mass or more relative to the overall amount of the cosmetic.

3. The water-in-oil emulsion cosmetic according to claim 1, further comprising (C) an ultraviolet scattering agent.

4. The water-in-oil emulsion cosmetic according to claim 1, wherein the total blended amount of the component (A) and the component (C) is 15% by mass or more relative to the overall amount of the cosmetic.

5. The water-in-oil emulsion cosmetic according to claim 1, further comprising (D) an oil phase thickener that contains:
(D-1) an organically modified clay mineral; and
(D-2) one or more types of oil gelling agents selected from among dextrin fatty acid esters, hydrocarbon-styrene copolymer gelling agents, polymer compounds having urethane backbones, glyceryl fatty acid esters, amino acid gelling agents and sucrose fatty acid esters.

6. The water-in-oil emulsion cosmetic according to claim 1, wherein the component (B) does not exhibit flowability at ambient temperature, and has a melting point of 30 °C to 60 °C at 1 atm.

7. The water-in-oil emulsion cosmetic according to claim 1, wherein the blended amount of the component (B) is 1% to 5% by mass relative to the overall amount of the cosmetic.

8. The water-in-oil emulsion cosmetic according to claim 1, having a viscosity of 10,000 mPa·s or higher.
